# EUROPÄISCHE PATENTSCHRIFT

(11) **EP 2 174 591 B1**
(45) Veröffentlichungstag und Bekanntmachung des Hinweises auf die Patenterteilung: **16.01.2019**
(21) Anmeldenummer: 08017714.0
(22) Anmeldetag: 09.10.2008
(51) Int. Cl.: A61B 5/15

(54) **Stechgerät**
Puncture instrument
Appareil de perçage

(43) Veröffentlichungstag der Anmeldung: 14.04.2010
(73) Patentinhaber: Roche Diabetes Care GmbH, 68305 Mannheim (DE); F. Hoffmann-La Roche AG, 4070 Basel (CH)
(72) Erfinder: Weiss, Thomas, 68307 Mannheim (DE); Gentsch, Susanne, 68519 Vrienheim (DE); Kolonko, Lydia, 64646 Heppenheim (DE); Forster, Richard, D-92269 Fensterbach (DE); Gorshöfer, Andreas, D-92449 Steinberg am See (DE); Ebert, Karl-Peter, D-64407 Fränkisch-Crumbach (DE); Wessel, Robert, D-29690 Schwarmstedt (DE)
(74) Vertreter: Twelmeier Mommer & Partner

(56) Entgegenhaltungen:
- WO-A-03/022130
- US-A1- 2005 125 019
- US-A1- 2006 155 215

## Beschreibung

Die Erfindung geht aus von einem Stechgerät mit den im Oberbegriff des Anspruchs 1 angegebenen Merkmalen. Ein derartiges Stechgerät ist aus der US 2005/125019 A1 und der US 2006/155215 A1 bekannt.

Um für analytisch-diagnostische Zwecke eine geringe Menge Körperflüssigkeit, beispielsweise Blut und/oder interstitielle Flüssigkeit, aus einem Körperteil, beispielsweise einem Finger, zu entnehmen, werden Lanzetten verwendet, die zur Erzeugung einer Stichwunde mit einem Stechgerät in das entsprechende Körperteil gestochen werden.

Um eine effiziente und möglichst schmerzarme Probengewinnung zu ermöglichen, lässt sich bei Stechgeräten üblicherweise die Stechtiefe einstellen und somit an die individuellen Erfordernisse eines Benutzers anpassen. Die Stechtiefe sollte nämlich einerseits groß genug sein, um eine ausreichend große Menge Körperflüssigkeit zu liefern, andererseits so gering wie möglich sein, um unnötige Schmerzen zu vermeiden.

Aus der EP 1 427 327 B1 ist ein Stechgerät bekannt, bei dem die Position eines Anschlags im Geräteinneren einstellbar ist, der eine Stichbewegung abstoppt und so den bei einer Stichbewegung auftretenden Hub auf das für eine gewünschte Stechtiefe erforderliche Maß begrenzt. Nachteilig hieran ist, dass beim Abstoppen einer Lanzette durch einen Anschlag Vibrationen entstehen können, die zu schmerzhaften Querbewegungen der Lanzette führen.

Aus der US 6 022 366 ist ein Stechgerät bekannt, bei dem zur Einstellung der Stechtiefe die Länge eines Lanzettenhalters angepasst werden kann. Hierfür muss jedoch eine Gerätekappe abgenommen werden, was für viele Benutzer mühsam ist. Zudem besteht dabei die Gefahr, dass sich ein Benutzer an einer Lanzette in dem Gerät verletzt.

Aus der US 2007/0055298 A1 ist ein Stechgerät bekannt, bei dem zur Stechtiefeneinstellung eine aufgeschraubte Endkappe verwendet wird. Diese Endkappe bildet ein Auflageelement, das eine Öffnung zum Anlegen an einen Körperteil aufweist, in dem eine Stichwunde erzeugt werden soll. Durch Drehen der aufgeschraubten Kappe bewegt sich diese in Stichrichtung relativ zu dem Stechantrieb des Geräts, so dass sich eine gewünschte Stechtiefe einstellen lässt. Nachteilig hieran ist jedoch, dass sich zwischen der Kappe und dem Gerätegehäuse ein Spalt bildet, wenn die Kappe zur Reduktion der Stechtiefe durch eine Schraubbewegung in Stichrichtung von dem Gehäuse wegbewegt wird. Ein solcher Spalt ist schwer zu reinigen. Zudem sammelt sich darin leicht Schmutz an.

Aufgabe der vorliegenden Erfindung ist es, einen Weg aufzuzeigen, wie sich ohne die vorstehend genannten Nachteilen bei einem Stechgerät eine Einstellbarkeit der Stechtiefe verwirklichen lässt.

Diese Aufgabe wird erfindungsgemäß durch ein Stechgerät mit den im Anspruch 1 angegebenen Merkmalen gelöst. Vorteilhafte Weiterbildungen der Erfindung sind Gegenstand der Unteransprüche.

Bei einem erfindungsgemäßen Stechgerät wird eine Einstellung der Stechtiefe durch eine Änderung der Position des Auflageelements relativ zu dem Stechantrieb erreicht. Vorteilhaft kann deshalb unabhängig von der gewünschten Stechtiefe bei jedem Lanzettenstich derselbe Hub ausgeführt werden. Ein vorzeitiges Abstoppen einer Lanzette, das schmerzhafte Vibrationen verursachen könnte, lässt sich somit vermeiden.

Das Auflageelement eines erfindungsgemäßen Stechgeräts ist mittels eines Gewindes drehbar und in Stichrichtung beweglich an einem Zwischenstück befestigt, das relativ zu dem Gehäuse drehbar beweglich ist. Eine Einstellung einer gewünschten Stechtiefe lässt sich deshalb selbst für Benutzer, deren manuelle Beweglichkeit durch Alter oder Krankheit eingeschränkt ist, mühelos vornehmen, indem das Zwischenstück relativ zu dem Auflageelement gedreht wird. Diese Drehbewegung bewirkt eine Bewegung des Auflageelements in Stichrichtung relativ zu dem Stechantrieb. Als Folge dieser Bewegung ändert sich der Abstand des Auflageelements von dem Stechantrieb und somit die eingestellte Stechtiefe. Bevorzugt bewirkt die Drehbewegung zugleich auch eine Änderung des Abstandes des Auflageelements von dem Gehäuse. Prinzipiell ist es jedoch auch möglich den Stechantrieb beweglich in dem Gehäuse zu montieren, so dass sich bei der Drehbewegung der Abstand des Auflageelements von dem Gehäuse nicht ändern muss.

Durch das erfindungsgemäße Zwischenstück lässt sich vorteilhaft vermeiden, dass zwischen dem Auflageelement und dem Gehäuse ein schwer zu reinigender Spalt entsteht, in dem sich Schmutz ansammeln könnte.

Eine Drehbewegung des Auflageelements relativ zu dem Gehäuse wird durch eine Führung verhindert. Indem eine Drehbewegung des Auflageelements relativ zu dem Gehäuse des Stechgeräts verhindert wird, behält das Auflageelement und insbesondere dessen Öffnung stets dieselbe Orientierung in Bezug auf den Stechantrieb des Stechgeräts bei. Dies ist für Stechgeräte, die mit einem mehrere Lanzetten enthaltenden Trommelmagazin arbeiten wichtig, da für einen Stich jeweils die Öffnung des Auflageelements fluchtend mit der jeweils an den Lanzettenantrieb angekoppelten Lanzette des Trommelmagazins positioniert sein muss.

Die Führung kann beispielsweise ein sich in Stichrichtung erstreckendes Führungselement aufweisen, das zwischen zwei Anschlägen angeordnet ist, gegenüber denen es in Stichrichtung beweglich ist. Das Führungselement kann beispielsweise als eine Nase oder Feder an dem Auflageelement ausgebildet sein. Möglich ist es auch, dass die Anschläge an dem Auflageelement ausgebildet sind und ein starr an dem Gehäuse angebrachtes Führungselement zwischen diese Anschläge hineinragt.

Ein Stechgerät mit einem Aufnahmefach für ein Trommelmagazin ist beispielsweise aus der EP 1 669 028 A1 bekannt, die diesbezüglich durch Bezugnahme zum Gegenstand der vorliegenden Anmeldung gemacht wird, insbesondere hinsichtlich möglicher Ausgestaltungen eines Trommelmagazins und eines mit ihm zusammenwirkenden Stechantriebs. Bevorzugt ist das Aufnahmefach in einer von dem Auflageelement und dem Zwischenstück gebildeten Stechtiefenverstelleinrichtung angeordnet.

Das Gewinde, mittels welchem das Auflageelement drehbar an dem Zwischenstück befestigt ist, kann an dem Auflageelement und/oder an dem Zwischenstück vorgesehen sein. Dabei genügt es, an einem dieser beiden Teile eine Nut oder Einkerbung vorzusehen, die ein Gewinde bildet. Als Gegengewinde genügt an sich ein einziger Vorsprung, der in dieses Gewinde eingreift. Um einem Verkippen und Klemmen vorzubeugen ist jedoch vorteilhaft, mehrere derartige Vorsprünge, insbesondere drei Vorsprünge vorzusehen, die beispielsweise als Gewindenocken in das Gewinde eingreifen.

Das Auflageelement sitzt zumindest zu einem Teil in dem Zwischenstück, das beispielsweise als eine Hülse ausgebildet sein kann. Je kleiner die eingestellte Stechtiefe ist, desto weiter ragt das Auflageelement aus dem Zwischenstück heraus. Indem das Auflageelement von dem Zwischenstück umgeben ist, lässt sich das Stechgerät besser reinigen. Bevorzugt ist dabei, dass das Auflageelement bei Einstellung einer minimalen Stichtiefe auf einem überwiegenden Teil seiner Länge von dem Zwischenstück umgeben ist.

Bevorzugt weist das Stechgerät eine Schiene zum Anstecken an ein Messgerät auf, wie es aus der EP 1 032 307 B1 bekannt ist. Ein Messgerät mit einem angesteckten Stechgerät bildet ein System, mit dem ein Benutzer eine Körperflüssigkeitsprobe gewinnen und auswerten kann. Ein solches System lässt sich vorteilhaft mit einer Hand bedienen, um durch einen Stich eine Probe zu gewinnen und anschließend auszuwerten, beispielsweise zur Messung der Glucosekonzentration.

Weitere Einzelheiten und Vorteile der Erfindung werden an einem Ausführungsbeispiel unter Bezugnahme auf die beigefügten Zeichnungen erläutert. Es zeigen:
- Figur 1a:: ein Ausführungsbeispiel eines erfindungsgemäßen Stechgeräts
- Figur 1b: ein Ausführungsbeispiel eines dazugehörenden Messgeräts;
- Figur 1c:: eine schematische Schnittansicht des Figur 1a gezeigten Stechgeräts mit daran befestigtem Messgerät;
- Figur 1d:: eine Explosionsansicht zu Figur 1a;
- Figur 2:: eine die Stechtiefenverstelleinrichtung des Stechgeräts;
- Figur 3:: eine Schnittansicht zu Figur 2;
- Figur 4:: eine Seitenansicht zu Figur 2;
- Figur 5:: eine Schnittansicht der Stechtiefenverstelleinrichtung bei eingefahrenem Auflageelement;
- Figur 6:: eine Seitenansicht zu Figur 5;
- Figur 7:: die Stechtiefenverstelleinrichtung in einer Explosionsdarstellung; und
- Figur 8:: eine Schnittansicht zu Figur 7.

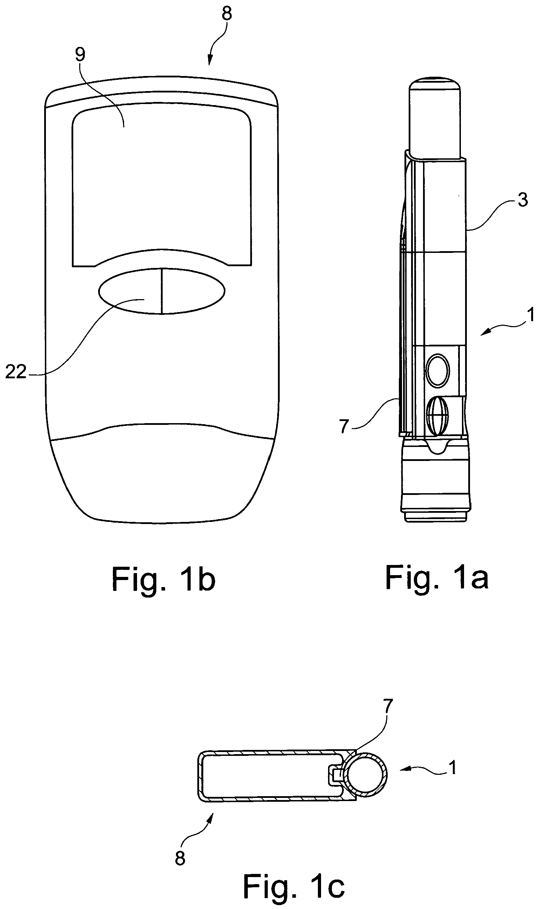

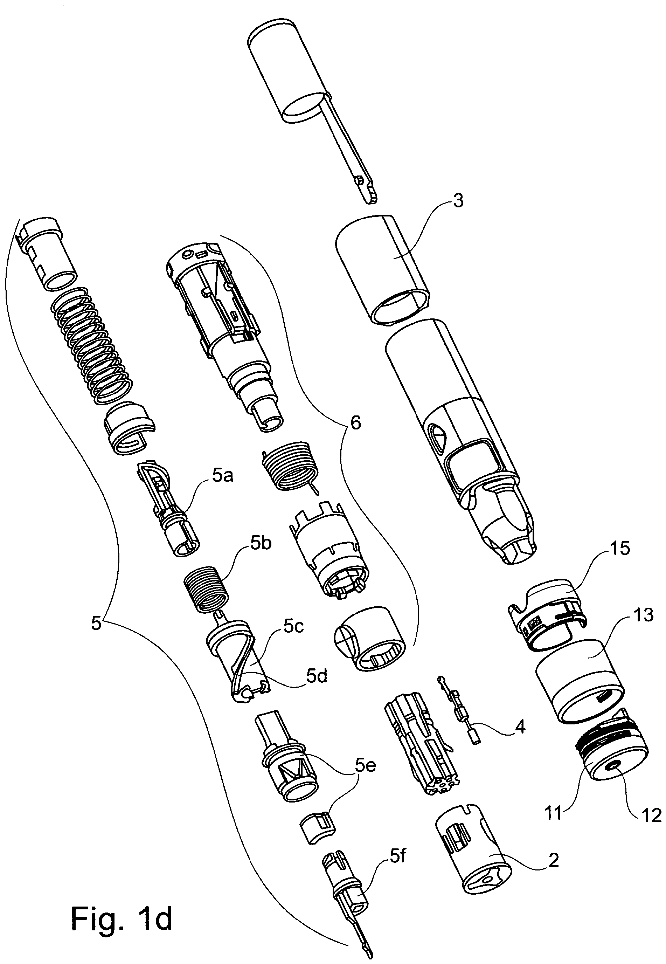

Die Figuren 1a und 1d zeigen ein Ausführungsbeispiel eines Stechgeräts 1 mit einem dazu gehörenden Trommelmagazin 2, in dem mehrere Lanzetten 4 enthalten sind. Das Stechgerät 1 hat ein Gehäuse 3, das einen in Figur 1a dargestellten Stechantrieb 5 umgibt. Geeignete Stechantriebe, mit denen sich Lanzetten in eine Stichbewegung versetzen lassen, sind im Stand der Technik hinlänglich bekannt und bedürfen deshalb an dieser Stelle keiner näheren Erläuterung. Besonders vorteilhaft lassen sich insbesondere Rotorantriebe verwenden, bei denen durch Drehen eines Spannelements 5a eine Antriebsfeder 5b gespannt wird, die bei einem Stich einen Antriebsrotor 5c in Drehung versetzt, der eine Steuerkurve 5d aufweist, die über einen Kopplungsmechanismus 5e eine Vorschubbewegung eines Lanzettenhalters 5f bewirkt, der eine Lanzette 4 des Trommelmagazins 2 vorschiebt und anschließend zurück zieht, so dass eine Stichwunde in einem an die Öffnung 12 des Geräts angelegten Körperteil erzeugt wird.

Das Stechgerät 1 hat ferner einen Fortschaltmechanismus 6, mit dem ein eingesetztes Trommelmagazin 2 schrittweise gedreht werden kann, so dass die in ihm ringförmig angeordneten Lanzetten 4 nacheinander fluchtend auf die Öffnung 12 ausgerichtet und für einen Stich verwendet werden können.

Das Stechgerät 1 hat an einer Seite eine Schiene 7, mit der es an einem in Figur 1b gezeigten Messgerät 8 befestigt werden kann. Figur 1c zeigt eine schematische Schnittansicht des Messgeräts 8 mit daran befestigtem Stechgerät 1. Wie man in Figur 1c sieht, wird das Stechgerät 1 zur Befestigung an des Messgerät 8 gesteckt, so dass die Schiene 7 in eine passende Ausnehmung des Messgeräts 8 eingreift.

Das Messgerät 8 hat eine Anzeigeeinrichtung 9 zum Anzeigen von Messergebnissen und Bedienungselemente 22 zur Betätigung. Das Messgerät 8 kann im übrigen wie handelsübliche Geräte zur Messung der Glucosekonzentration einer Blutprobe ausgebildet sein und beispielsweise mit Testelementen arbeiten, die eine photometrische oder elektrochemische Konzentrationsbestimmung ermöglichen. Indem das Stechgerät 1 an dem Messgerät 8 befestigt wird, entsteht ein System, das sich mit einer Hand bedienen lässt, um einen Stich zur Probengewinnung auszuführen und anschließend eine dadurch gewonnen Probe für eine Messung aufzunehmen.

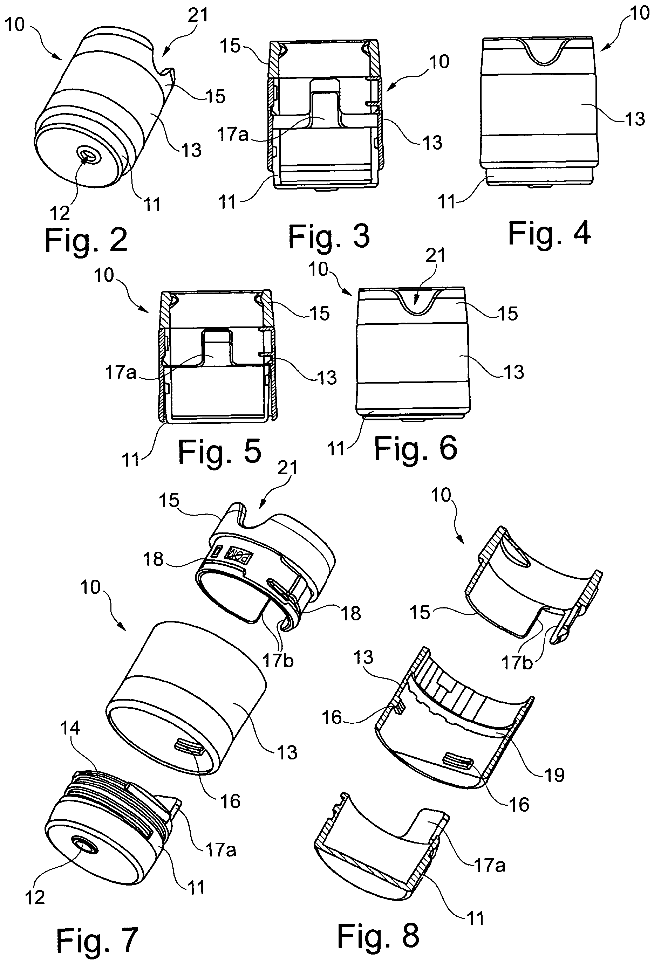

Die Besonderheit des dargestellten Stechgeräts 1 ist die in den Figuren 2 bis 8 dargestellte Stechtiefenverstelleinrichtung 10. Zu der Stechtiefenverstelleinrichtung 10 gehören ein Auflageelement 11 mit einer Öffnung 12 zum Anlegen an ein Körperteil, in dem eine Stichwunde erzeugt werden soll, und ein Zwischenstück 13, an dem das Auflageelement 11 mittels eines in Figur 7 dargestellten Gewindes 14 drehbar und in Stichrichtung beweglich befestigt ist. Vorteilhaft lässt sich auf diese Weise die Stechtiefe auch dann bequem einstellen, wenn das Stechgerät 1 an dem Messgerät 8 befestigt ist.

Bei dem dargestellten Ausführungsbeispiel ist das Zwischenstück 13 an einem Träger 15 befestigt, der von dem Gerätegehäuse 3 abnehmbar ist. Prinzipiell ist es jedoch auch möglich, den Träger 15 in das Gehäuse 3 zu integrieren und das Zwischenstück 13 direkt an dem Gehäuse 3 zu befestigen.

Eine Drehbewegung des Zwischenstücks 13 relativ zu dem Auflageelement 11 bewirkt wegen des zwischen diesen beiden Teilen wirkenden Gewindes 14, dass das Auflageelement 11 in Stichrichtung bewegt wird. Da der Stechantrieb bei jeder Stichbewegung denselben Lanzettenhub bewirkt, ergibt sich bei einem erhöhten Abstand des Auflageelements 11 von dem Gehäuse 3 bzw. dem Träger 15 eine reduzierte Stechtiefe. Wird dagegen der Abstand zwischen dem Auflageelement 11 und dem Gehäuse 3 bzw. dem Träger 15 reduziert, also das Auflageelement 11 tiefer in das Zwischenstück 13 hineingeschraubt, ergibt sich eine erhöhte Stechtiefe. In dem Figuren 2 bis 4 ist die Stechtiefenverstelleinrichtung 10 in einer Einstellung für eine geringe Stechtiefe, in den Figuren 5 und 6 in einer Einstellung für eine hohe Stechtiefe gezeigt.

Bei dem dargestellten Ausführungsbeispiel umfasst das zwischen dem Auflageelement 11 und dem Zwischenstück 13 wirkende Gewinde 14 eine Nut in dem Auflageelement 11. In diese Nut greifen Vorsprünge 16 an der Innenseite des Zwischenstücks 15 ein. Prinzipiell wäre es jedoch auch möglich, entsprechende Vorsprünge 16 an dem Auflageelement 11 vorzusehen und das Zwischenstück 15 mit einem vollständigen Gewinde auszustatten.

Die Stechtiefenverstelleinrichtung 10 hat eine Linearführung 17a, 17b die eine Drehbewegung des Auflageelements 11 relativ zu dem Träger 15 und somit auch relativ zu dem Gehäuse 3 verhindert. Diese Führung 17a, 17b wird von einem sich in Stichrichtung erstreckenden Führungselement 17a gebildet, das nach Art einer Nut-und-Federverbindung zwischen zwei Anschläge 17b eingereift, gegenüber denen es in Stichrichtung beweglich ist. Bei dem dargestellten Ausführungsbeispiel ist das Führungselement 17a als eine Nase ausgebildet, die in eine passende Ausnehmung des Trägers 15 eingreift.

Die Führung 17a, 17b bewirkt, dass die azentrisch angeordnete Öffnung 12 des Auflageelements 11 unabhängig von der eingestellten Stechtiefe stets ihre Orientierung in Bezug auf das Gehäuse 3 und somit in Bezug auf den darin enthaltenen Stechantrieb beibehält und folglich die jeweils zum Gebrauch vorgesehene Lanzette eines eingesetzten Trommelmagazins fluchtend auf die Öffnung 12 ausgerichtet ist.

Das als Hülse ausgebildete Zwischenstück 13 ist mit dem ringförmig ausgebildeten Träger 15 verrastet. Diese Verrastung geschieht bei dem dargestellten Ausführungsbeispiel durch Eingriff eines oder mehrerer Vorsprünge 18 des Trägers 15 in eine Ringnut 19 des Zwischenstücks 13. Auf diese Weise ist das Zwischenstück 13 gegenüber dem Träger 15 drehbar und gleichzeitig in axialer Richtung fixiert. Möglich ist es auch, das Zwischenstück13 mittels eines weiteren Gewindes drehbar mit dem Gehäuse zu verbinden. Vorzugsweise ist in diesem Fall das Gewinde, mittels welchem das Auflageelement 11 drehbar an dem Zwischenstück 13 befestigt ist, gegenläufig zu dem zweiten Gewinde ist, mittels welchem das Zwischenstück 13 drehbar mit dem Gehäuse 3, beispielsweise über den Träger 15, verbunden ist.

Der Träger 15 ist von dem Gehäuse 3 des Stechgeräts 1 abnehmbar, um ein Trommelmagazin 2 in einem dafür vorgesehenen Aufnahmefach des Stechgeräts 1 auszutauschen. Bei dem dargestellten Ausführungsbeispiel ist das Trommelmagazin 2 in der von dem Träger 15, dem Zwischenstück 13 und dem Auflageelement 11 gebildeten Stechtiefenverstelleinrichtung angeordnet.

Bei dem dargestellten Ausführungsbeispiel verrastet der Träger 15 mit dem Gehäuse 3 und hat hierfür entsprechende Rastelemente 20, die bei dem dargestellten Ausführungsbeispiel als Ausnehmungen ausgebildet sind, in die passende Rastelemente des Gehäuses 3 eingreifen. An seiner dem Gehäuse 3 zugewandten Unterseite weist der Träger 15 eine Einbuchtung 21 auf, in die ein Fixierelement des Gehäuses 3 eingreift, um den Träger 15 verdrehsicher in einer vorbestimmten Position an dem Gehäuse 3 zu halten.

**Bezugszahlen**
- 1: Stechgerät
- 2: Trommelmagazin
- 3: Gehäuse
- 4: Lanzette
- 5: Stechantrieb
- 5a: Spannelement
- 5b: Antriebsfeder
- 5c: Antriebsrotor
- 5d: Steuerkurve
- 5e: Kopplungsmechanismus
- 5f: Lanzettenhalter
- 6: Fortschaltmechanismus
- 7: Schiene
- 8: Messgerät
- 9: Anzeigeeinrichtung
- 10: Stechtiefenverstelleinrichtung
- 11: Auflageelement
- 12: Öffnung
- 13: Zwischenstück
- 14: Gewinde
- 15: Träger
- 16: Vorsprung
- 17a: Führung
- 17b: Führung
- 18: Vorsprünge
- 19: Ringnut
- 20: Rastelement
- 21: Einbuchtung
- 22: Bedienungselemente

## Patentansprüche

1. Stechgerät mit
einem Stechantrieb (5), um eine Lanzette (4) in eine Stichbewegung zu versetzen,
einem Gehäuse (3), das den Stechantrieb (5) umgibt, und
einem Auflageelement (11) mit einer Öffnung (12) zum Anlegen an einen Körperteil, in dem eine Stichwunde erzeugt werden soll,
wobei das Auflageelement (11) zur Einstellung der Stechtiefe in Stichrichtung relativ zu dem Stechantrieb (5) beweglich ist,
wobei das Auflageelement (11) mittels eines Gewindes (14) drehbar und in Stichrichtung beweglich an einem Zwischenstück (13) befestigt ist, das relativ zu dem Gehäuse (3) drehbar beweglich ist, wobei eine Führung (17a, 17b) eine Drehbewegung des Auflageelements (11) relativ zu dem Gehäuse (3) verhindert,
**dadurch gekennzeichnet, dass** das Auflageelement (11) mindestens zu einem Teil in dem Zwischenstück (13) sitzt.

2. Stechgerät nach Anspruch 1, **dadurch gekennzeichnet, dass** die Führung (17a, 17b) ein sich in Stichrichtung erstreckendes Führungselement (17a, 17b) aufweist, das zwischen zwei Anschlägen angeordnet ist, gegenüber denen es in Stichrichtung beweglich ist.

3. Stechgerät nach Anspruch 2, **dadurch gekennzeichnet, dass** das Führungselement (17a, 17b) als eine Nase an dem Auflageelement (11) ausgebildet ist.

4. Stechgerät nach einem der vorstehenden Ansprüche, **dadurch gekennzeichnet, dass** das Gewinde (14) eine Nut in dem Auflageelement (11) umfasst.

5. Stechgerät nach Anspruch 4, **dadurch gekennzeichnet, dass** das Zwischenstück (13) an einer Innenfläche wenigstens einen Vorsprung (16), vorzugsweise wenigstens drei Vorsprünge (16), aufweist, welche in die Nut eingreifen.

6. Stechgerät nach einem der vorstehenden Ansprüche **dadurch gekennzeichnet, dass** das Zwischenstück (13) eine Hülse ist.

7. Stechgerät nach einem der vorstehenden Ansprüche, **dadurch gekennzeichnet, dass** das Gehäuse (3) einen abnehmbaren Träger (15) aufweist, mit dem das Zwischenstück (13) verrastet ist.

8. Stechgerät nach Anspruch 7, **gekennzeichnet durch** ein Aufnahmefach für ein Trommelmagazin (2), wobei sich das Aufnahmefach durch Abnehmen des Trägers (15) für einen Austausch eines Trommelmagazins (2) öffnen lässt.

9. Stechgerät nach Anspruch 8, **dadurch gekennzeichnet, dass** das Aufnahmefach in einer von dem Träger (15), dem Zwischenstück (13) und dem Auflageelement (11) gebildeten Stechtiefenverstelleinrichtung (10) angeordnet ist.

10. Stechgerät nach einem der Ansprüche 7 bis 9, **dadurch gekennzeichnet, dass** der Träger (15) verdrehsicher mit dem Gehäuse (3) verrastet.

11. Stechgerät nach einem der Ansprüche 7 bis 10, **dadurch gekennzeichnet, dass** der Träger (15) an seiner dem Gehäuse (3) zugewandten Unterseite eine Einbuchtung (21) aufweist, in die ein Fixierelement des Gehäuses (3) eingreift.

12. Stechgerät einem der vorstehenden Ansprüche, **dadurch gekennzeichnet, dass** das Auflageelement (11) eine Schraubkappe ist.

13. Stechsystem mit einem Stechgerät (1) nach einem der vorstehenden Ansprüche und einem Trommelmagazin (2), das mehrere Lanzetten (4) enthält.

## Claims

1. Lancing device comprising
a lancet drive (5) to cause a lancet (4) to effect a puncturing movement,
a housing (3) that encloses the lancet drive (5), and
a touching element (11) with an opening (12) for applying a part of a body in which a prick wound is to be produced,
the touching element (11) being movable for the purpose of adjusting the puncturing depth in a puncturing direction relative to the lancet drive (5), wherein the touching element (11) is rotatable by means of a screw thread and movably in the puncturing direction fixed to an intermediate piece (13) that is rotatable relative to the housing (3), wherein a guide (17a, 17b) prevents rotation of the touching element (11) relative to the housing (3), **characterized in that** the touching element (11) is arranged at least in part inside the intermediate piece (13).

2. The lancing device as defined in Claim 1, **characterized in that** the guide (17, 17b) comprises a guide element (17a, 17b) that extends in the puncturing direction and is arranged between two stops relative to which it is movable in the puncturing direction.

3. The lancing device as defined in Claim 2, **characterized in that the** guide element (17a, 17b) is configured as a lug disposed on the touching element (11).

4. The lancing device as defined in any of the preceding claims, **characterized in that** the thread (14) comprises a groove in the touching element (11).

5. The lancing device as defined in Claim 4, **characterized in that** at least one projection (16), preferably at least three projections (16), engaging the groove, are disposed on an inner surface of the intermediate piece (13).

6. The lancing device as defined in any of the preceding claims, **characterized in that** the intermediate piece (13) is a sleeve.

7. The lancing device as defined in any of the preceding claims, **characterized in that** the housing (3) comprises a detachable carrier on which the intermediate piece (13) is locked in position.

8. The lancing device as defined in Claim 7, **characterized by** a receptacle for a rotary magazine (2) which receptacle can be opened for replacement of a rotary magazine (2) by removing the carrier (15).

9. The lancing device as defined in Claim 8, **characterized in that** the receptacle is arranged in a lancing device adjusting means (10) constituted by the carrier (15), the intermediate piece (13) and the touching element (11).

10. The lancing device as defined in any of Claims 7 to 9, **characterized in that the** carrier (15) is secured against rotation and locked in position on the housing (3).

11. The lancing device as defined in Claims 7 to 10, **characterized in that** the bottom surface of the carrier (15), facing the housing (3), exhibits an indentation (21) that is engaged by a fixing element disposed on the housing (3).

12. The lancing device as defined in any of the preceding claims, **characterized in that** the touching element (11) is a screw cap.

13. Lancing system having a lancing device (1) according to any of the preceding claims and a rotary magazine (2) containing a plurality of lancets (4).

## Revendications

1. Appareil de perçage avec
un mécanisme d'entraînement de perçage (5) pour déplacer une lancette (4) dans un mouvement de piqûre,
un boîtier (3) qui entoure le mécanisme d'entraînement de perçage (5), et un élément d'appui (11) avec une ouverture (12) pour une mise en place au niveau d'une partie du corps dans laquelle une plaie par piqûre doit être générée,
dans lequel l'élément d'appui (11) est mobile pour le réglage de la profondeur de perçage dans la direction de piqûre par rapport au mécanisme d'entraînement de perçage (5),
dans lequel l'élément d'appui (11) est fixé au moyen d'un filetage (14) de manière rotative et mobile dans la direction de piqûre au niveau d'une pièce intermédiaire (13) qui est mobile de manière rotative par rapport au boîtier (3), dans lequel un guidage (17a, 17b) empêche un mouvement de rotation de l'élément d'appui (11) par rapport au boîtier (3),
**caractérisé en ce que** l'élément d'appui (11) est posé au moins en partie dans la pièce intermédiaire (13).

2. Appareil de perçage selon la revendication 1, **caractérisé en ce que** le guide (17a, 17b) présente un élément de guidage (17a, 17b) qui s'étend dans la direction de piqûre, lequel élément de guidage est disposé entre deux butées, par rapport auxquelles il est mobile dans la direction de piqûre.

3. Appareil de perçage selon la revendication 2, **caractérisé en ce que** l'élément de guidage (17a, 17b) est réalisé en tant qu'embout au niveau de l'élément d'appui (11).

4. Appareil de perçage selon l'une des revendications précédentes, **caractérisé en ce que** le filetage (14) comprend une rainure dans l'élément d'appui (11).

5. Appareil de perçage selon la revendication 4, **caractérisé en ce que** la pièce intermédiaire (13) présente au niveau d'une surface interne au moins une saillie (16), de préférence au moins trois saillies (16), lesquelles s'emboîtent dans la rainure.

6. Appareil de perçage selon l'une des revendications précédentes, **caractérisé en ce que** la pièce intermédiaire (13) est une douille.

7. Appareil de perçage selon l'une des revendications précédentes, **caractérisé en ce que** le boîtier (3) présente un support amovible (15) avec lequel la pièce intermédiaire (13) est encliquetée.

8. Appareil de perçage selon la revendication 7, **caractérisé par** un compartiment pour un chargeur de type tambour (2), dans lequel le compartiment est ouvert par le biais du retrait du support (15) pour un remplacement d'un chargeur de type tambour (2).

9. Appareil de perçage selon la revendication 8, **caractérisé en ce que** le compartiment est disposé dans un dispositif de réglage de profondeur de perçage (10) formé par le support (15), la pièce intermédiaire (13) et l'élément d'appui (11).

10. Appareil de perçage selon l'une des revendications 7 à 9, **caractérisé en ce que** le support (15) est encliqueté avec le boîtier (3) de manière à ne pas entrer en rotation.

11. Appareil de perçage selon l'une des revendications 7 à 10, **caractérisé en ce que** le support (15) présente au niveau de sa face inférieure tournée vers le boîtier (3) une échancrure (21) dans laquelle s'emboîte un élément de fixation du boîtier (3).

12. Appareil de perçage selon l'une des revendications précédentes, **caractérisé en ce que** l'élément d'appui (11) est un bouchon à vis.

13. Système de perçage avec un appareil de perçage (1) selon l'une des revendications précédentes et un chargeur de type tambour (2) qui contient plusieurs lancettes (4).
